# EUROPEAN PATENT SPECIFICATION

(11) **EP 1 728 563 B1**
(45) Date of publication and mention of the grant of the patent: **04.08.2021**
(21) Application number: 06011014.5
(22) Date of filing: 29.05.2006
(51) Int. Cl.: B06B 1/06, G10K 11/30

(54) **ULTRASONIC PROBE AND ULTRASONIC PROBE MANUFACTURING METHOD**
ULTRASCHALLSONDE UND VERFAHREN ZUR HERSTELLUNG DER ULTRASCHALLSONDE
SONDE À ULTRASONS ET MÉTHODE DE FABRICATION D'UNE TELLE SONDE

(30) Priority: 30.05.2005 JP 2005157460
(43) Date of publication of application: 06.12.2006
(73) Proprietor: Toshiba Medical Systems Corporation, Otawara-Shi, Tochigi-Ken 324-8550 (JP)
(72) Inventor: Takeuchi, Takashi IPD Toshiba Medical Systems Corp, Otawara-shi Tochigi 324-8550 (JP); Shikata, Hiroyuki IPD Toshiba Medical Systems Corp, Otawara-shi Tochigi 324-8550 (JP)
(74) Representative: Kramer Barske Schmidtchen Patentanwälte PartG mbB

(56) References cited:
- GB-A- 2 086 269
- JP-A- 1 027 400
- US-A- 5 711 058
- US-A- 5 743 855
- US-A1- 2001 041 837

## Description

The present invention relates to an ultrasonic probe used in an ultrasonic diagnosis apparatus, an ultrasonic flaw detection apparatus, and the like, and a manufacturing method therefor.

An ultrasonic probe is a device for applying ultrasonic waves to a target and receiving reflected waves from interfaces with different acoustic impedances in the target in order to, for example, image the interior of the target. As ultrasonic image apparatuses using such an ultrasonic probe, a medical diagnosis apparatus for examining the interior of a human body, an examination apparatus targeted to flaw detection in a metal weld, and the like exist.

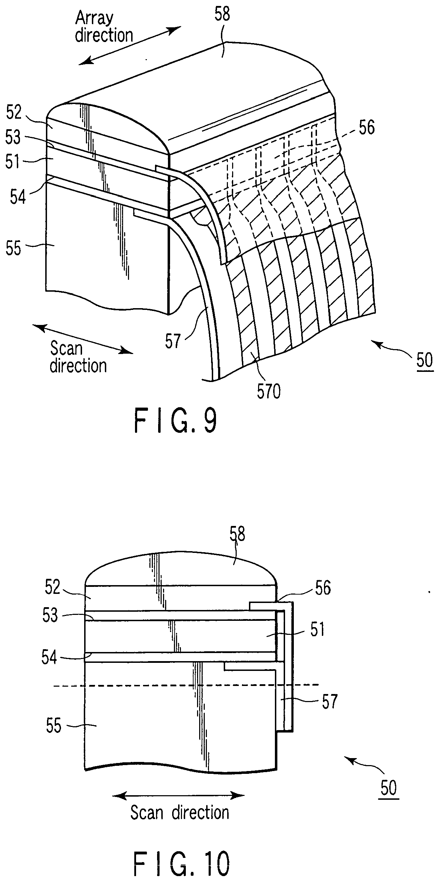

This ultrasonic probe mainly comprises a piezoelectric element. FIGS. 9 and 10 are views for explaining the typical arrangement of an ultrasonic probe 50. Referring to FIGS. 9 and 10, an acoustic matching layer 52 is formed on the ultrasonic wave transmission/reception surface of an ultrasonic transducer 51. An acoustic lens 58 is formed on an entire acoustic matching layer 52. A backing member 55 is formed on the lower surface of the ultrasonic transducer 51 (the opposite surface to the ultrasonic wave transmission/reception surface) to hold it.

A GND common electrode plate 56 is connected to a first electrode 53 of the ultrasonic transducer 51. An electrical signal lead for ultrasonic wave transmission/reception is extracted from a second electrode 54 of the ultrasonic transducer 51 and is connected to a flexible printed wiring board 57. This lead is connected to the pulser and receiver of the ultrasonic diagnosis apparatus through a signal cable (not shown).

A conventional ultrasonic probe is completed by performing each of the following processing. First of all, the flexible printed wiring board 57 and a piezoelectric element (i.e., an ultrasonic transducer block before cutting) are joined to each other, and a flexible printed wiring board portion protruding from the piezoelectric element is bent to the backing member side at an angle of about 90° before dicing. The resultant structure is then diced to split the piezoelectric element for each CH, thereby forming each ultrasonic transducer 51. If the transducer to be formed is to have an aspect ratio of 0.6 or more, a better vibration mode can be obtained by sub-dicing each 1-ch portion. A convex scanning ultrasonic probe is primarily fixed to a flexible backing member (not shown), and is then split for each ultrasonic transducer 51 up to the backing member by dicing in the same manner as described above. Subsequently, the flexible backing member is joined to the R-shaped backing member 55, thereby forming an ultrasonic probe.

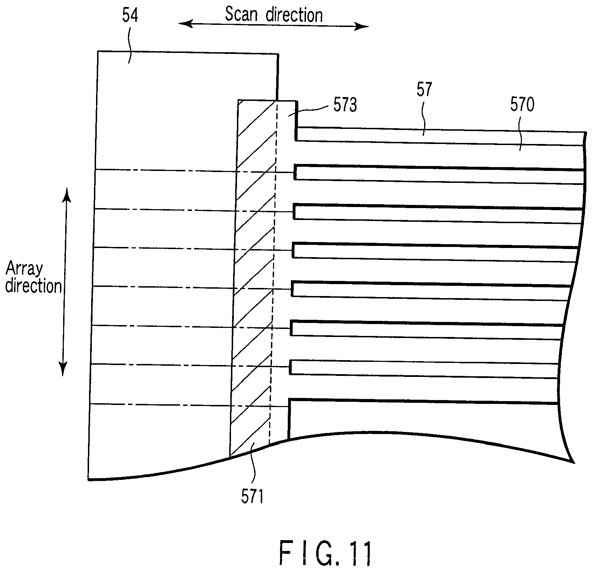

As shown in FIG. 11, the flexible printed wiring board 57 used for a conventional ultrasonic probe includes a common electrode 571 made of a copper foil, individual electrodes 570, and auxiliary electrode portions 573. The flexible printed wiring board 57 is joined to part (the area indicated by the broken line) of the second electrode 54 by soldering part of the common electrode 571, and is cut together with the piezoelectric element such that the common electrode 571 is reliably cut.

Ultrasonic probes manufactured by the conventional technique described above may lack in reliability in terms of durability, acoustic characteristics, and the like. For example, as shown in FIG. 11, the joint portion between the flexible printed wiring board 57 and each ultrasonic transducer has only a width equal to the width of the common electrode 571. That is, a sufficiently reliable joint area cannot be ensured. In addition, since the common electrode 571 must be reliably cut, the electrode must be cut deep to the backing member. For this reason, a flexible backing member for primary fixing is required in addition to an R-shaped backing member, and warpage occurs in the slice direction due to strong restoring force against the R-bend of these members. As a result, a slice acoustic field shift occurs, and acoustic characteristics vary.

Ultrasonic probes manufactured by the conventional technique are low in the machinability of ultrasonic transducers. This causes variation in acoustic characteristics. For example, as shown in FIG. 11, the piezoelectric element is split into the ultrasonic transducers by cutting the piezoelectric element along the scan direction (elevation direction), together with the common electrode 571 (see the one-dot dashed line in FIG. 11). In this case, a target on which a cutting load discontinuously exists in the cutting direction is cut, and the machinability deteriorates. Since the common electrode 571 for soldering is a copper foil, in particular, the cutting load is large, and the machinability greatly deteriorates.

US 5,711,058 relates to probes used in ultrasonic imaging of the human anatomy, in particular, to ultrasonic transducer arrays for use in electronic beam images to make wide field-off-view scans.

JP 01-027400 relates to an ultrasonic probe.

US 5,743,855 relates to a broadband phased array transducer design with frequency controlled two dimension capability and methods for manufacture thereof.

US 2001/0041837 A1 relates to an ultrasonic probe and a method of manufacturing the same.

GB 2 086 269 A relates to a method of producing an acoustic transducer.

The present invention has been made in consideration of the above situation, and has as its object to provide an acoustically stable ultrasonic probe with high reliability and manufacturing method therefor.

This object is achieved by an ultrasonic probe according to claim 1 or a manufacturing method according to claim 4.

Further improvements are given in the dependent claims.

The invention can be more fully understood from the following detailed description when taken in conjunction with the accompanying drawings, in which:
FIG. 1 is a perspective view showing an outer appearance of an ultrasonic probe 10 according to an embodiment;
FIG. 2 is a side view of the ultrasonic probe 10 according to this embodiment;
FIG. 3 is a view for explaining the arrangement of a flexible printed wiring board 17;
FIG. 4 is a view for explaining the arrangement of the flexible printed wiring board 17;
FIG. 5 is a flowchart showing a sequence of manufacturing the ultrasonic probe 10;
FIG. 6 is a view showing how an ultrasonic transducer block (ultrasonic transducer 11) and the flexible printed wiring board 17 are joined to each other;
FIG. 7 is a view for explaining a modification of the ultrasonic probe 10 according to this embodiment;
FIG. 8 is a view for explaining another modification of the ultrasonic probe 10 according to this embodiment;
FIG. 9 is a perspective view for explaining the arrangement of a conventional ultrasonic probe 50;
FIG. 10 is a view for explaining the arrangement of the conventional ultrasonic probe 50; and
FIG. 11 is a view for explaining a cutting step in manufacturing the conventional ultrasonic probe 50.

An embodiment of the present invention will be described below with reference to the views of the accompanying drawing. Note that the same reference numerals in the following description denote constituent elements having substantially the same functions and arrangements, and a repetitive description will be made only when required.

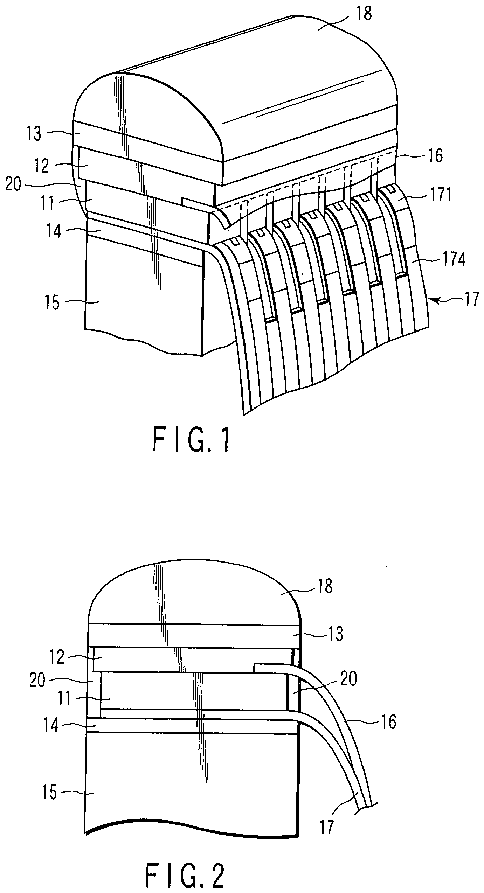

The arrangement of an ultrasonic probe according to the embodiment of the present invention will be described first with reference to FIG. 1.

FIG. 1 is a view showing the outer appearance of an ultrasonic probe 10 according to this embodiment. As shown in FIG. 1, the ultrasonic probe 10 comprises ultrasonic transducers 11, first acoustic matching layer 12, second acoustic matching layer 13, lining layer 14, backing member 15, GND common electrode 16, flexible printed wiring board 17, acoustic lens 18, and side-surface resin layer 20.

Each ultrasonic transducer 11 has upper-side and lower-side electrodes (not shown) provided on a two- or three-component piezoelectric ceramics member shaped in a rectangular form. A plurality of such transducers are arrayed to form a convex surface in the ultrasonic wave application direction. The ultrasonic transducer 11 generates an ultrasonic wave on the basis of a driving signal from the pulser and converts a reflected wave from an object into an electrical signal.

The first acoustic matching layer 12 and the second acoustic matching layer 13 are provided on the ultrasonic wave application side of the ultrasonic transducers 11. The acoustic impedances between the object and the ultrasonic transducers 11 can be matched by adjusting physical parameters such as sound speed, thicknesses, and acoustic impedances in the first acoustic matching layer 12 and the second acoustic matching layer 13.

Although the ultrasonic probe 10 according to this embodiment is configured to have the first acoustic matching layer 12 and the second acoustic matching layer 13, the probe may be configured to have only the first acoustic matching layer 12. The width of the first acoustic matching layer 12 in the scan direction is larger than that of the ultrasonic transducer 11, and the width of the second acoustic matching layer 13 in the scan direction is larger than that of the first acoustic matching layer 12. Such an arrangement is used to reduce the load applied when a blade is pulled out in the dicing step for ultrasonic transducers (to be described later).

The lining layer 14 is provided on the lower side of the flexible printed wiring board 17 (on the opposite side to the side where ultrasonic waves are applied). The lining layer 14 is provided to ensure a sufficient depth for dicing in the dicing step for the ultrasonic transducer (to be described later), and is cut halfway by the blade in the thickness direction (i.e., is not completely cut). In addition, this layer is made of a resin such as polyimide to ensure proper acoustic matching between the flexible printed wiring board 17 and the ultrasonic transducer 11.

The backing member 15 is provided on the lower side of the lining layer 14 (the opposite side to the side where ultrasonic waves are applied) to mechanically support the ultrasonic transducer 11 and damp the ultrasonic transducer 11 so as to shorten ultrasonic pulses. The thickness of the backing member 15 is set to be sufficient for the wavelength of ultrasonic waves to be used (i.e., a thickness that sufficiently attenuates ultrasonic waves in the direction of the backing) in order to maintain good acoustic characteristics. Likewise, in consideration of acoustic matching, an inorganic substance is mixed in the backing member 15 to have an acoustic impedance within ±20% of the acoustic impedance of the lining layer 14.

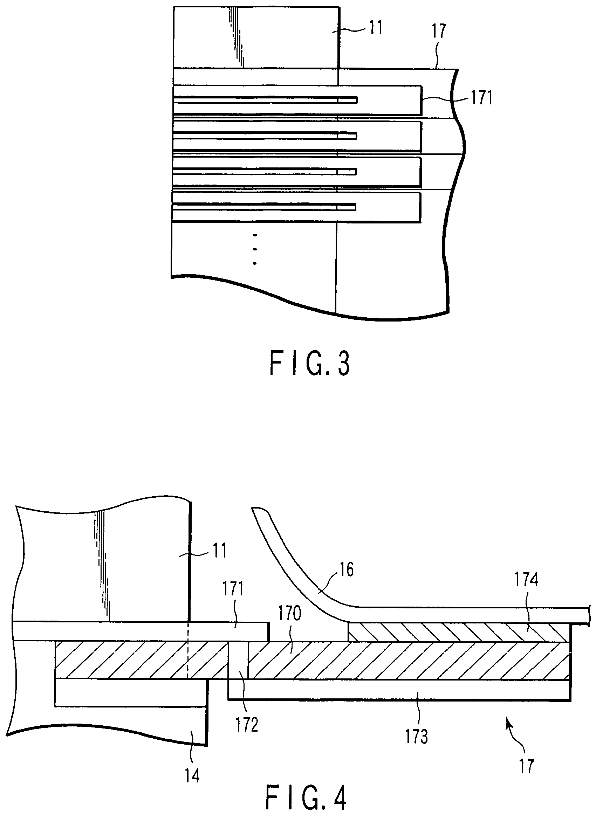

The GND common electrode 16 is provided on one end of an upper-side (ultrasonic wave application side) electrode (not shown) of each ultrasonic transducer 11. This electrode is used for the ground connection of the upper-side electrodes. This electrode is extracted from an end portion of each ultrasonic transducer 11 as shown in FIGS. 1 and 2, and is connected halfway to the flexible printed wiring board 17 to be integrated, as shown in FIG. 4.

The flexible printed wiring board 17 is provided on the lower side of each ultrasonic transducer 11, and comprises a wiring pattern made of a copper foil for applying power to each ultrasonic transducer 11, a flexible base member, and the like.

FIGS. 3 and 4 are views for explaining the arrangement of the flexible printed wiring board 17. As shown in FIGS. 3 and 4, the flexible printed wiring board 17 includes a base member 170 comprising a resin such as polyimide, first wiring portions 171, second wiring portions 172, third wiring portions 173, and fourth wiring portions 174.

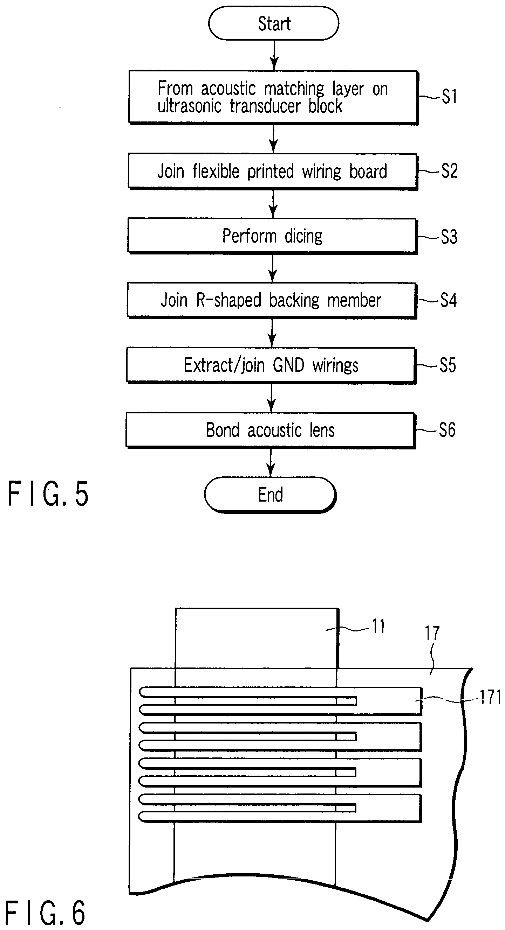

The first wiring portion 171 is connected to the lower-side electrode of each ultrasonic transducer 11, and is used to extract an electric wiring from the lower-side electrode. As shown in FIG. 3 (or FIG. 6), the first wiring portion 171 bifurcates into two branches at the join portion with the ultrasonic transducer 11, and the branches are combined for each channel outside the joint portion. In addition, the first wiring portions 171 are arranged at predetermined intervals so as not be cut in the dicing step for the ultrasonic transducers 11 (to be described later).

As shown in FIG. 4, each third wiring portion 173 is provided on the opposite surface to the first wiring portion 171, and electrically connects the ultrasonic diagnosis apparatus body to the ultrasonic probe 10. Each second wiring portion 172 extends through the base member 170 and electrically connects the first wiring portion 171 to the third wiring portion 173. Each fourth wiring portion 174 is provided on the upper surface of the flexible printed wiring board 17 (on the same side as the first wiring portion 171) and is connected to the GND common electrode 16.

The acoustic lens 18 is made of silicone rubber or the like which has an acoustic impedance similar to that of a living body. This lens converges ultrasonic beams by using acoustic refraction and improves the resolution.

The side-surface resin layer 20 is a resin layer for filling the steps between the first acoustic matching layer 12 and the ultrasonic transducers 11 in the scan direction and between the second acoustic matching layer 13 and the first acoustic matching layer 12 in the scan direction. This layer comprises, for example, a filler-containing adhesive.

A manufacturing method for the ultrasonic probe 10 having the above arrangement will be described next with reference to FIGS. 5 and 6. FIG. 5 is a flowchart showing a sequence of manufacturing the ultrasonic probe 10. As shown in FIG. 5, first of all, the first acoustic matching layer 12 and the second acoustic matching layer 13 are formed on the upper-side electrode of a block (to be referred to as an "ultrasonic transducer block" hereinafter) obtained by forming upper-side and lower-side electrodes for voltage application on a piezoelectric element (step S1). Note that the first acoustic matching layer 12 and the second acoustic matching layer 13 are formed such that the first acoustic matching layer 12 has a width larger than that of the ultrasonic transducer block in the scan direction, and the second acoustic matching layer 13 has a width larger than that of the first acoustic matching layer 12 in the scan direction.

As shown in FIG. 6, the first wiring portions 171 of the flexible printed wiring board 17 are jointed to the lower-side electrode of the ultrasonic transducer block (ultrasonic transducers 11) (step S2). The ultrasonic transducer block is diced at predetermined intervals by using the blade (step S3). This dicing is executed such that the blade passes through between the patterns of the first wiring portions 171 shown in FIGS. 3 and 6, and the lining layer 14 is cut to a certain depth to reliably cut the first acoustic matching layer 12, second acoustic matching layer 13, and ultrasonic transducer block. Therefore, the blade does not cut the wiring patterns made of a hard copper foil. For this reason, the load applied to the blade in cutting operation is continuous, and are lower than those in the prior art.

Since the width of the first acoustic matching layer 12 is larger than the width of the ultrasonic transducer 11 in the scan direction on both sides, and the width of the second acoustic matching layer 13 is larger than the width of the first acoustic matching layer 12 in the scan direction on both sides, the blade is sequentially pulled out, starting from the ultrasonic transducer 11 in cutting operation. Sequentially pulling out the blade from the target to be cut in this manner makes it possible to disperse the load applied to the blade.

In order to array the ultrasonic transducers 11 in a convex shape with respect to the ultrasonic wave application surface, the R-shaped backing member 15 is joined (step S4), and a GND wiring portion is extracted from the upper-side electrode of each ultrasonic transducer 11 by using the GND common electrode 16 (step S5). Subsequently, the acoustic lens 18 is bonded on the second acoustic matching layer 13 (step S6), thereby completing the ultrasonic probe 10.

### (Embodiment)

An embodiment of the ultrasonic probe 10 will be described next. This embodiment is directed to a 3.5-MHz convex scanning ultrasonic probe. The thickness of an ultrasonic transducer block to be used is about 350 µm, and a sub-dice is required to obtain an effective longitudinal vibration mode.

The flexible printed wiring board 17 has a composite structure having thin copper foils (i.e., the first wiring portions 171, third wiring portions 173, and fourth wiring portions 174) formed on both sides of the base member 170 made of polyimide, and the second wiring portions 172 as through holes. The array pitch of the first wiring portions 171 is 400 µm, and the interval between the adjacent first wiring portions 171 (i.e., the width of the base without any wiring pattern on the transducer lower surface) is 80 µm. The first wiring portions 171 are combined for each channel outside the transducer lower surface.

Note that since the base member is bent when it is housed, it is preferably thin. In consideration of the relationship between the depth of dicing and the acoustic matching layer on the lower surface of the transducer 11, the 100-µm thick lining layer 14 made of polyimide resin is formed on the opposite surface of the base member to the surface to which the ultrasonic transducer 11 is boded.

A manufacturing method for the convex scanning ultrasonic probe 10 will be described next with reference to FIG. 5.

First of all, the first acoustic matching layer 12 and the second acoustic matching layer 13 are formed on the ultrasonic transducer block (step S1), and the flexible printed wiring board 17 is joined to the ultrasonic transducer block with a conductive adhesive or the like (step S2).

The ultrasonic transducer block to which the flexible printed wiring board 17 is joined is temporarily fixed as work on a cutting base made of glass or the like, and is diced at 200-µm intervals by a 50-µm blade (step S3). At this time, the blade passes through the 80-µm wide base member areas between the first wiring portions 171, and cuts into the transducer to a depth of 50 µm from the transducer lower surface in consideration of acoustic matching.

The temporarily fixed work, i.e., the ultrasonic transducer block to which the flexible printed wiring board 17 is joined, is detached, and the R-shaped backing member 15 is joined to the lining layer 14 (step S4). In this case, the R-shaped backing member 15 preferably has the same acoustic impedance as that of the lining layer 14 made of polyimide and the base member of the flexible printed wiring board 17. For this reason, the acoustic impedance of the backing member 15 is adjusted to a value within ±20% of the acoustic impedance of the a polyimide base by adding a desired amount of ZnO powder to chloroprene rubber.

A GND wiring is extracted from the upper-side electrode of each ultrasonic transducer 11 with the GND common electrode 16 (step S5). Thereafter, the acoustic lens 18 is bonded onto the second acoustic matching layer 13 (step S6), thereby completing the 3.5-MHz convex scanning ultrasonic probe 10.

According to the above arrangement, the following effects can be obtained.

The first wiring portions 171 extracted from the lower-side electrodes of the ultrasonic transducers 11 are arrayed at intervals larger than the width of the blade to be used in the dicing step, and the blade passes through the base member areas between the first wiring portions 171. Therefore, the cutting load in the cutting direction (scan direction) can be made uniform, and there is no need to cut any copper foil requiring a large cutting load. This makes it possible to improve the machinability and reduce variations in acoustic characteristics as compared with the prior art, thereby providing a highly reliable ultrasonic probe.

In consideration of the fact that the blade is generally of a disc type, this ultrasonic probe is configured such that the widths of the ultrasonic transducer 11, first acoustic matching layer 12, and second acoustic matching layer 13 increase to the upper side (ultrasonic wave application side) in the order named. In the cutting step, the blade is pulled out from the ultrasonic transducer 11, first acoustic matching layer 12, and second acoustic matching layer 13 in the order named. Therefore, the cutting load can be dispersed, and the machinability can be improved as compared with the prior art in which a load is applied at once.

The lower-side electrodes of the ultrasonic transducers 11 and the flexible printed wiring board 17 are joined with the first wiring portions 171 throughout the lower surfaces of the ultrasonic transducers 11. Therefore, a sufficient joint area can be ensured, and suitable signal transmission/reception and durability can be ensured. The lower-side electrodes and the first wiring portions 171, in particular, are joined throughout the entire width in the scan direction of the ultrasonic transducer 11, and hence the ultrasonic transducers 11 have substantially the same strength at their lower surfaces. As compared with the conventional ultrasonic probe having the arrangement in which the backing member 55 and the electrode 571 having different hardnesses are joined to the ultrasonic transducer lower surface as shown in FIGS. 9 to 11, for example, the durability in the ultrasonic wave transmission/reception direction (i.e., a direction almost perpendicular to the array direction and the scan direction) can be improved.

Such a joint form eliminates the necessity of the auxiliary electrode portions 573 for joining, which is aimed at removing the join portions between the lower-side electrodes of the ultrasonic transducers 51 and the flexible printed wiring board 57 from the effective apertures as much as possible as in the conventional ultrasonic probe shown in FIG. 11. As a consequence, as compared with the conventional ultrasonic probe, unnecessary apertures other than the effective apertures can be reduced, and a compact ultrasonic probe with a small living body contact portion can be realized.

In manufacturing the ultrasonic probe 10, there is no need to cut the common electrode 571 shown in FIG. 11, and hence there is no need to use any flexible backing member for primarily fixing the piezoelectric element to be cut and the flexible printed wiring board. As compared with the prior art, therefore, the thickness of the backing member can be reduced, and the restoring force with respect to the R-bend of the backing member can be reduced. As a consequence, the warpage of the ultrasonic transducer 11 array in the slice direction can be prevented, and a slice acoustic field shift and acoustic characteristic variations can be prevented.

In addition, the backing member of this ultrasonic probe is configured to have an acoustic impedance almost equal to that of the lining layer 14 made of polyimide. This makes it possible to keep the acoustic matching property with the lining layer 14 and cause reception ultrasonic waves to be efficiently transmitted/attenuated backward from the ultrasonic transducers 11.

Conventionally, the depth of cutting in a cutting step for an ultrasonic transducer is influenced by joining shifts between the transducers and the flexible printed wiring board. Such joining shifts cause variations in cutting depth. As a result, the flexible backing member for primary fixing which has an acoustic matching function on the backing side varies in thickness, and acoustic characteristics vary. In contrast to this, in the cutting step in manufacturing the ultrasonic probe 10, the ultrasonic transducer block, the fourth wiring portion 174 of the flexible printed wiring board 17, and the lining layer 14, which have relatively good machinability, are cut halfway. Therefore, there is no need to cut the common electrode 571 shown in FIG. 11, and the cutting depth does not depend on joining shifts between transducers and a flexible printed wiring board unlike in the prior art. In addition, the thickness of the backing member becomes uniform. As a consequence, the acoustic characteristics can be improved as compared with the prior art.

While the present invention has been described on the basis of the embodiment of the present invention, it will be obvious to those skilled in the art that various changes and modifications may be made thereto without departing from the scope of the present invention as defined by the appended claims.

For example, as will be described next, the embodiments can be variously modified.

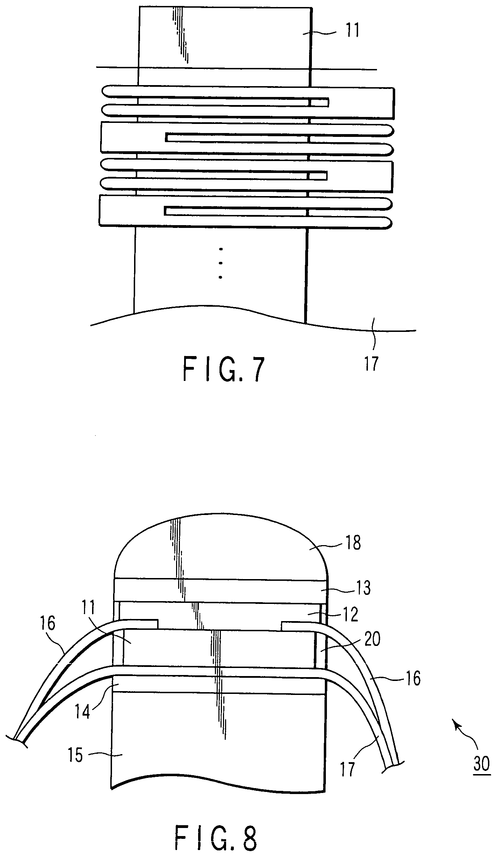

As shown in FIGS. 1 and 2, the above embodiment has exemplified the ultrasonic probe in which the signal wirings are extracted from the ultrasonic transducers 11 to one side in the scan direction by using the first wiring portions 171 and the GND common electrode 16. In contrast, signal wirings from the ultrasonic transducers 11 may be at least alternately extracted to both sides in the scan direction. In this case, for example, using the flexible printed wiring board 17 shown in FIG. 7 makes it possible to manufacture an ultrasonic probe 30 having signal wirings extracted from the ultrasonic transducers 11 to both sides as shown in FIG. 8. In this case, as shown in FIG. 8, it is also preferable that the GND common electrode 16 be extracted to both sides in the scan direction together with the first wiring portions 171.

The scope of the present invention is defined by the appended claims.

## Claims

1. An ultrasonic probe, comprising:
a plurality of ultrasonic transducers (11) which are arranged in an array form along a first direction and each include a first electrode and a second electrode;
a flexible printed wiring board (17) including a base member (170) and a plurality of first wiring portions (171) provided on a first surface of the base member (170), the plurality of first wiring portions (171) being connected to the second electrodes of said respective ultrasonic transducers (11);
a lining layer (14), and
a backing member (15) joined to the lining layer (14),
**characterized in that**:
the lining layer (14) is provided on a second surface of the base member (170), wherein the lining layer is provided between the second surface of the base member (170) and the backing member (15), and is made of a resin which ensures acoustic matching between the flexible printed wiring board (17) and the plurality of ultrasonic transducers (11);
the backing member (15) is configured to attenuate ultrasonic waves applied by the plurality of ultrasonic transducers (11) ;
a plurality of recesses, which divide said plurality of ultrasonic transducers (11) in an array form, extend through the flexible printed wiring board (17), and reach to a certain depth into the lining layer (14); and
said plurality of first wiring portions are arrayed along the first direction at intervals larger than array intervals of said plurality of ultrasonic transducers (11), and each has a width smaller than a width of said each ultrasonic transducer in the first direction.

2. The probe according to claim 1, wherein said plurality of first wiring portions (171) are connected to said respective second electrodes throughout the entire width of said each ultrasonic transducer in a scan direction.

3. The ultrasonic probe according to claim 1 or claim 2, wherein the plurality of recesses reach halfway into the lining layer (14).

4. A manufacturing method for an ultrasonic probe including a plurality of ultrasonic transducers (11) arrayed at predetermined intervals along a first direction, the method comprising:
joining ultrasonic transducer blocks and a flexible printed wiring board (17), the ultrasonic transducer blocks including first electrodes and second electrodes, and the flexible printed wiring board (17) including a base member (170) and a plurality of first wiring portions (170) provided on a first surface of the base member (170), the plurality of first wiring portions (171) being arrayed along the first direction at intervals larger than the predetermined intervals, and each having a width smaller than a width of said each ultrasonic transducer in the first direction;
connecting the plurality of first wiring portions (171) to the second electrodes of said respective ultrasonic transducer blocks;
forming a lining layer (14) on a second surface of the base member (170), the lining layer (14) being made of a resin which ensures acoustic matching between the flexible printed wiring board (17) and the plurality of ultrasonic transducers (11);
extracting said plurality of ultrasonic transducers (11) by cutting the ultrasonic transducer block and the flexible printed wiring board (17) at the area of the base member (170) between the adjacent first wiring portions (171) and forming recesses reaching to a certain depth into the lining layer (14) through the ultrasonic transducer block and the flexible printed wiring board (17); and
joining a backing member (15) to the lining layer (14) , the backing member (15) being configured to attenuate ultrasound waves to be applied by the plurality of ultrasonic transducers (11) .

5. The method according to claim 4, wherein in extracting said each ultrasonic transducer (11), part of the lining layer (14) is cut in a thickness direction.

6. The method according to claim 5, wherein
the recesses reach halfway into the lining layer (14).

## Patentansprüche

1. Ultraschallsensor mit:
mehreren Ultraschallwandlern (11), die entlang einer ersten Richtung in einer Reihe angeordnet sind und jeweils eine erste Elektrode und eine zweite Elektrode aufweisen;
einer flexiblen Leiterplatte (17) mit einem Basisbauteil (170) und mehreren ersten Verdrahtungsteilen (171), die auf einer ersten Oberfläche des Basisbauteils (170) vorgesehen sind, wobei die mehreren ersten Verdrahtungsteile (171) mit den zweiten Elektroden der jeweiligen Ultraschallwandler (11) verbunden sind;
einer Zwischenschicht (14) und
einem Trägerbauteil (15), das mit der Zwischenschicht (14) verbunden ist,
**dadurch gekennzeichnet, dass**:
die Zwischenschicht (14) auf einer zweiten Oberfläche des Basisbauteils (170) vorgesehen ist, wobei die Zwischenschicht zwischen der zweiten Oberfläche des Basisbauteils (170) und dem Trägerbauteil (15) vorgesehen ist und aus einem Harz besteht, das eine akustische Anpassung zwischen der flexiblen Leiterplatte (17) und den mehreren Ultraschallwandlern (11) sicherstellt;
das Trägerbauteil (15) zum Dämpfen von Ultraschallwellen, die durch die mehreren Ultraschallwandler (11) aufgebracht werden, ausgebildet ist;
sich mehrere Vertiefungen, die die mehreren Ultraschallwandler (11) in eine Reihe unterteilen, durch die flexible Leiterplatte (17) erstrecken und eine bestimmte Tiefe in der Zwischenschicht (14) erreichen; und
die mehreren ersten Verdrahtungsteile entlang der ersten Richtung bei Intervallen, die größer als Anordnungsintervalle der mehreren Ultraschallwandler (11) sind, angeordnet sind und jeweils eine Breite aufweisen, die kleiner ist als eine Breite des jeweiligen Ultraschallwandlers in der ersten Richtung.

2. Sensor nach Anspruch 1, bei dem die mehreren ersten Verdrahtungsteile (171) über die gesamte Breite des jeweiligen Ultraschallwandlers in einer Abtastrichtung mit den jeweiligen zweiten Elektroden verbunden sind.

3. Ultraschallsensor nach Anspruch 1 oder Anspruch 2, bei dem die mehreren Vertiefungen bis zur Hälfte der Zwischenschicht (14) reichen.

4. Herstellungsverfahren für einen Ultraschallsensor mit mehreren Ultraschallwandlern (11), die bei vorbestimmten Intervallen entlang einer ersten Richtung angeordnet sind, mit folgenden Schritten:
Verbinden von Ultraschallwandlerblöcken und einer flexiblen Leiterplatte (17), wobei die Ultraschallwandlerblöcke erste Elektroden und zweite Elektroden aufweisen und die flexible Leiterplatte (17) ein Basisbauteil (170) und mehrere erste Verdrahtungsteile (170), die auf einer ersten Oberfläche des Basisbauteils (170) vorgesehen sind, aufweist, wobei die mehreren ersten Verdrahtungsteile (171) entlang der ersten Richtung bei Intervallen, die größer als die vorbestimmten Intervalle sind, angeordnet sind und jeweils eine Breite aufweisen, die kleiner ist als eine Breite des jeweiligen Ultraschallwandlers in der ersten Richtung;
Verbinden der mehreren ersten Verdrahtungsteile (171) mit den zweiten Elektroden der jeweiligen Ultraschallwandlerblöcke;
Ausbilden einer Zwischenschicht (14) auf einer zweiten Oberfläche des Basisbauteils (170), wobei die Zwischenschicht (14) aus einem Harz besteht, das eine akustische Anpassung zwischen der flexiblen Leiterplatte (17) und den mehreren Ultraschallwandlern (11) sicherstellt;
Extrahieren der mehreren Ultraschallwandler (11) durch Schneiden des Ultraschallwandlerblocks und der flexiblen Leiterplatte (17) bei dem Bereich des Basisbauteils (170) zwischen den benachbarten ersten Verdrahtungsteilen (171) und Ausbilden von Vertiefungen, die eine bestimmte Tiefe in der Zwischenschicht (14) erreichen, durch den Ultraschallwandlerblock und die flexible Leiterplatte (17); und
Verbinden eines Trägerbauteils (15) mit der Zwischenschicht (14), wobei das Trägerbauteil (15) zum Dämpfen von Ultraschallwellen, die durch die mehreren Ultraschallwandler (11) aufzubringen sind, ausgebildet ist.

5. Verfahren nach Anspruch 4, bei dem das Extrahieren des jeweiligen Ultraschallwandlers (11) ein Schneiden eines Teils der Zwischenschicht (14) in einer Dickenrichtung beinhaltet.

6. Verfahren nach Anspruch 5, bei dem
die Vertiefungen bis zur Hälfte der Zwischenschicht (14) reichen.

## Revendications

1. Sonde à ultrasons, comprenant :
une pluralité de transducteurs ultrasonores (11) qui sont agencés sous forme de réseau le long d'une première direction et qui comprennent chacun une première électrode et une seconde électrode ;
une carte de câblage imprimé flexible (17) comprenant un élément de base (170) et une pluralité de premières parties de câblage (171) prévues sur une première surface de l'élément de base (170), la pluralité de premières parties de câblage (171) étant connectées aux secondes électrodes desdits transducteurs ultrasonores respectifs (11) ;
une couche de revêtement (14), et
un élément de support (15) relié à la couche de revêtement (14),
**caractérisé en ce que**
la couche de revêtement (14) est prévue sur une seconde surface de l'élément de base (170), dans lequel la couche de revêtement est prévue entre la seconde surface de l'élément de base (170) et l'élément de support (15), et est faite d'une résine qui assure une adaptation acoustique entre la carte de câblage imprimé flexible (17) et la pluralité de transducteurs ultrasonores (11) ;
l'élément de support (15) est configuré pour atténuer les ondes ultrasonores appliquées par la pluralité de transducteurs ultrasonores (11) ;
une pluralité d'évidements, qui divisent ladite pluralité de transducteurs ultrasonores (11) sous la forme d'un réseau, s'étendent à travers la carte de câblage imprimé flexible (17), et atteignent une certaine profondeur dans la couche de revêtement (14) ; et
ladite pluralité de premières parties de câblage sont disposées le long de la première direction à des intervalles plus grands que les intervalles de réseau de ladite pluralité de transducteurs ultrasonores (11) et chacune a une largeur plus petite qu'une largeur dudit chaque transducteur ultrasonore dans la première direction.

2. Sonde selon la revendication 1, dans laquelle ladite pluralité de premières parties de câblage (171) sont connectées auxdites secondes électrodes respectives sur toute la largeur dudit chaque transducteur ultrasonore dans une direction de balayage.

3. Sonde à ultrasons selon la revendication 1 ou la revendication 2, dans laquelle la pluralité d'évidements atteint la moitié de la couche de revêtement (14).

4. Procédé de fabrication d'une sonde à ultrasons comprenant une pluralité de transducteurs ultrasonores (11) disposés en réseau à des intervalles prédéterminés le long d'une première direction, le procédé comprenant les étapes consistant à :
joindre des blocs de transducteurs ultrasonores et une carte de câblage imprimé flexible (17), les blocs de transducteurs ultrasonores comprenant des premières électrodes et des secondes électrodes, et la carte de câblage imprimé flexible (17) comprenant un élément de base (170) et une pluralité de premières parties de câblage (171) prévues sur une première surface de l'élément de base (170), la pluralité de premières parties de câblage (171) étant disposées le long de la première direction à des intervalles plus grands que les intervalles prédéterminés, et chacune ayant une largeur plus petite qu'une largeur dudit chaque transducteur ultrasonore dans la première direction ;
connecter la pluralité de premières parties de câblage (171) aux secondes électrodes desdits blocs de transducteurs ultrasonores respectifs ;
former une couche de revêtement (14) sur une seconde surface de l'élément de base (170), la couche de revêtement (14) étant constituée d'une résine qui assure une adaptation acoustique entre la carte de câblage imprimé flexible (17) et la pluralité de transducteurs ultrasonores (11) ;
extraire ladite pluralité de transducteurs ultrasonores (11) en coupant le bloc de transducteur ultrasonore et la carte de câblage imprimé flexible (17) dans la zone de l'élément de base (170) entre les premières parties de câblage adjacentes (171) et en formant des évidements atteignant une certaine profondeur dans la couche de revêtement (14) à travers le bloc de transducteur ultrasonore et la carte de câblage imprimé flexible (17) ; et
joindre un élément de support (15) à la couche de revêtement (14), l'élément de support (15) étant configuré pour atténuer les ondes ultrasonores devant être appliquées par la pluralité de transducteurs ultrasonores (11).

5. Procédé selon la revendication 4, dans lequel lors de l'extraction dudit chaque transducteur ultrasonore (11), une partie de la couche de revêtement (14) est découpée dans le sens de l'épaisseur.

6. Procédé selon la revendication 5, dans lequel les évidements atteignent la moitié de la couche de revêtement (14).
